(19) 

Europäisches
Patentamt

European
Patent Office

Office européen
des brevets

(11) **EP 4 302 774 A1**

(12) **EUROPEAN PATENT APPLICATION**
published in accordance with Art. 153(4) EPC

(43) Date of publication:
**10.01.2024 Bulletin 2024/02**

(21) Application number: **21928681.2**

(22) Date of filing: **20.05.2021**

(51) International Patent Classification (IPC):
**A61K 38/17** *(2006.01)*    **A61K 48/00** *(2006.01)*
**A61P 9/00** *(2006.01)*    **A61P 37/02** *(2006.01)*

(52) Cooperative Patent Classification (CPC):
**A61K 38/17; A61K 48/00; A61P 7/10; A61P 9/00;**
**A61P 9/10; A61P 11/00; A61P 29/00; A61P 31/14;**
**A61P 37/02; C12Q 1/6883; G01N 33/68;**
**Y02A 50/30**

(86) International application number:
**PCT/CN2021/094932**

(87) International publication number:
**WO 2022/183600 (09.09.2022 Gazette 2022/36)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **02.03.2021 CN 202110231913**

(71) Applicant: **Shanghai Children's Medical Center,**
**Shanghai**
**Jiaotong University School of Medicine**
**Shanghai 200127 (CN)**

(72) Inventors:
• **ZHANG, Zhen**
**Shanghai 200127 (CN)**
• **ZHANG, Min**
**Shanghai 200127 (CN)**

• **WANG, Wenfeng**
**Shanghai 200127 (CN)**
• **DING, Xiaoning**
**Shanghai 200127 (CN)**
• **WANG, Ye**
**Shanghai 200127 (CN)**
• **YANG, Junjie**
**Shanghai 200127 (CN)**
• **WEI, Lu**
**Shanghai 200127 (CN)**

(74) Representative: **Lavoix**
**Bayerstraße 83**
**80335 München (DE)**

Remarks:
The complete document including Reference
Table(s) and the Sequence Listing(s) can be
downloaded from the EPO website

(54) **USE OF ENHANCED TBX1 EXPRESSION IN REPAIRING HEART TISSUE INJURY**

(57) Use of an active ingredient in repairing heart tissue injury. Specifically, disclosed is an active ingredient capable of being used for repairing heart tissue injury and treating diseases related to lymphatic abnormalities, comprising: a TBX1 protein, a coding gene thereof, or an accelerator thereof, or a combination thereof. The TBX1 protein can promote the proliferation of heart lymphatic endothelium cells, play an immunoregulatory role, promote the establishment of an immunosuppressive microenvironment in the myocardium after heart tissue injury, inhibit autoimmune responses after the heart tissue injury, relieve concurrent inflammation of the heart tissue, and promote the repair and regeneration of the injured heart tissue.

EP 4 302 774 A1

**Description**

**TECHNICAL FIELD**

The present invention relates to the field of treatment of cardiovascular disease, particularly the use of TBX1 in repairing cardiac tissue injury.

**BACKGROUND**

Cardiovascular disease is one of the leading causes in China, and the mortality rate has been increasing in recent years. Myocardial cells in mammals can only proliferate for a short period after birth. Once the myocardium matures, it exits the cell cycle, making myocardial damage typically irreversible and associated with poor prognosis. This can lead to serious consequences; for instance, up to 60% of ischemic myocardial infarction patients develop heart failure within six years of their initial event. Current treatments for myocardial infarction primarily focus on restoring blood flow to reduce myocardial ischemic injury and regulating neural and humoral mechanisms through medication to slow adverse ventricular remodeling. However, these treatments cannot entirely prevent ventricular remodeling after myocardial infarction and a considerable number of patients progress to heart failure within a short period even without ongoing ischemic injury.

Therefore, there is an urgent need in this field to develop new therapies for heart diseases.

**SUMMARY OF INVENTION**

The purpose of this invention is to provide an active ingredient for effectively treating heart diseases and/or lymphatic vessel abnormalities associated with cardiac injury and the use thereof.

In the first aspect of the invention, it provides a use of an active ingredient for preparing a formulation or drug, which is used for:

   (i) activating proliferation of cardiac lymphatic endothelial cells;
   (ii) inhibiting cardiac autoimmune reactions;
   (iii) promoting the transformation of M1-type macrophages into M2-tpye macrophages;
   (iv) repairing cardiac injury;
   (v) treating heart diseases associated with cardiac injury;
   (vi) treating diseases associated with lymphatic abnormalities;

wherein, the active ingredient comprises TBX1 protein, an encoding sequence thereof, a promoting agent thereof, or a combination thereof.

In another preferred embodiment, the encoding sequence includes DNA, cDNA, mRNA.

In another preferred embodiment, "promoting the transformation of M1-type macrophages into M2-type macrophages" refers to promoting the transformation of M1-type macrophages (promoting inflammatory responses) into M2 macrophages (promoting tissue repair) in myocardial tissue.

In another preferred embodiment, "heart diseases associated with cardiac injury" refers to heart diseases related to immune responses, especially autoimmune responses.

In another preferred embodiment, the heart diseases associated with cardiac injury are selected from the group consisting of myocardial infarction, myocarditis, idiopathic dilated cardiomyopathy, Chagas' cardiomyopathy, rheumatic heart disease, heart damage caused by coronavirus disease 2019 (COVID-19), and a combination thereof.

In another preferred embodiment, the diseases associated with lymphatic abnormalities are selected from the group consisting of: primary lymphedema caused by lymphatic vessel developmental defects, secondary lymphedema caused by lymphatic vessel rupture or obstruction, Turner syndrome, yellow nail syndrome, Hennekam syndrome, and other genetic syndromes presenting with lymphatic vessel edema, and a combination thereof.

In the second aspect of the invention, it provides a use of TBX1 protein, an encoding sequence thereof, or a diagnostic reagent thereof for preparing a diagnostic reagent or diagnostic kit, which is used for prognostic assessment of heart diseases associated with cardiac injury or diseases associated with lymphatic abnormalities.

In another preferred embodiment, the diagnostic reagent is selected from the group consisting of primers, probes, chips, antibodies, and a combination thereof.

In another preferred embodiment, compared to normal individuals or controls, an increase in TBX1 expression level and/or activity suggests a favorable prognosis for the diagnosed subject.

In another preferred embodiment, compared to normal individuals or controls, a decrease in TBX1 expression level and/or activity suggests a poor prognosis for the diagnosed subject.

In another preferred embodiment, the diagnosed subject has or is suspected to have heart diseases associated with cardiac injury or diseases associated with lymphatic vessel abnormalities.

In another preferred embodiment, the method for prognostic assessment of heart diseases associated with cardiac injury or diseases associated with lymphatic abnormalities includes: providing a subject sample, detecting the expression level of TBX1 protein (T1) in the subject sample, and comparing it with the average expression level of TBX1 protein (T0) in cardiac lymphatic vessels of myocardial infarction patients;

if T1 is greater than T0, it indicates a favorable prognosis for the subject;
if T1 is less than T0, it indicates a poor prognosis for the subject.

In another preferred embodiment, the method for prognostic assessment of heart diseases associated with cardiac injury or diseases associated with lymphatic abnormalities includes providing a subject sample, and detecting whether the subject sample has TBX1 encoding and/or regulatory sequence variants. If the TBX1 variant leads to loss or reduction of TBX1 function, it suggests a poor prognosis for the subject. If the TBX1 variant enhances TBX1 function, it suggests a favorable prognosis for the subject.

In another preferred embodiment, the TBX1 function refers to promoting lymphatic vessel regeneration and forming an immunosuppressive microenvironment.

In another preferred embodiment, the heart diseases associated with cardiac injury include myocardial infarction.

In another preferred embodiment, the subject has a myocardial infarction.

In another preferred embodiment, the subject is a human or a non-human mammal.

In another preferred embodiment, the sample includes a cardiac tissue sample, a cardiac cell sample, a lymphatic endothelial cell sample, a lymph fluid sample, a peripheral blood cell sample, or a combination thereof.

In another preferred embodiment, the sample include a cell sample, a DNA sample, a RNA sample, or a combination thereof.

In another preferred embodiment, the kit further includes detection reagents for additional biomarkers detection, wherein the additional biomarkers are selected from the group consisting of: Top2a, Mki67, Cenpe, Aurkb, and a combination thereof.

In another preferred embodiment, if the expression levels (or abundance) of these additional biomarkers increase, it suggests that the TBX1 variant leads to enhanced TBX1 functionality.

In another preferred embodiment, compared to the normal population or control group, an increase in the expression levels (or abundance) of these additional biomarkers suggests a favorable prognosis for the diagnosed subject.

In another preferred embodiment, compared to the normal population or control group, a decrease in the expression levels (or abundance) of these additional biomarkers suggests a poor prognosis for the diagnosed subject.

In the third aspect of the present invention, it provides an active ingredient capable of being used for repairing cardiac injury and treating diseases associated with lymphatic abnormalities, wherein the active ingredient comprises TBX1 protein, an encoding sequence thereof, a promoting agent thereof, or a combination thereof.

In another preferred embodiment, the active ingredient is a promoting agent of TBX1 protein.

In another preferred embodiment, the promoting agent promotes the expression of TBX1 protein within the lymphatic vessels of the heart and other parts of the body.

In another preferred embodiment, the promoting agent upregulates the functionality of TBX1 protein within the lymphatic vessels of the heart and other parts of the body.

In the fourth aspect of the present invention, it provides an expression vector, wherein the expression vector contains an expression cassette for expressing the TBX1 protein.

In another preferred embodiment, the expression vector is selected from the group consisting of: DNA, RNA, a plasmid, an eukaryotic expression vector, a prokaryotic expression vector, a lentiviral vector, an adenoviral vector, an adeno-associated viral vector, a retroviral vector, a transposon, and a combination thereof.

In another preferred embodiment, the expression vector specifically transfects lymphatic endothelial cells.

In another preferred embodiment, the expression cassette has a structure shown in the Formula I from the 5' end to the 3' end:

$$Z0\text{-}Z1\text{-}Z2 \qquad (I)$$

wherein,

each "-" represents an independent chemical bond or nucleotide connecting sequence;
Z0 is absent or a 5' UTR sequence;
Z1 is a nucleotide sequence encoding the TBX1 protein; and

Z2 is absent or a 3' UTR sequence.

In another preferred embodiment, the expression cassette further includes a lymphatic endothelial-specific promoter operably linked to the nucleotide sequence encoding the TBX1 protein.

In another preferred embodiment, the length of each nucleotide connecting sequence is 1-30 nt, preferably 1-15 nt, and more preferably 3-6 nt.

In another preferred embodiment, the nucleotide sequence encoding the TBX1 protein is derived from humans or non-human mammals.

In another preferred embodiment, the nucleotide sequence is as shown in SEQ ID NO. 1.

In another preferred embodiment, the nucleotide sequence is as shown in SEQ ID NO. 2.

In the fifth aspect of the present invention, it provides a host cell containing the expression vector of the fourth aspect of the present invention.

In another preferred embodiment, the host cell is a eukaryotic cell or a prokaryotic cell.

In another preferred embodiment, the host cell is selected from the group consisting of: a bacterial cell, a yeast cell, and a mammalian cell.

In another preferred embodiment, the host cell is a lymphatic endothelial cell.

In another preferred embodiment, the host cell is a cardiac lymphatic endothelial cell.

In the sixth aspect of the present invention, it provides a pharmaceutical formulation comprising (a) the active ingredient of the third aspect of the present invention, or the expression vector of the fourth aspect of the present invention, or the host cell of the fifth aspect of the present invention, and (b) a pharmaceutically acceptable carrier, excipient, or diluent.

In another preferred embodiment, the pharmaceutical formulation is in the form of a lyophilized preparation, liquid preparation, or a combination thereof.

In another preferred embodiment, the pharmaceutical formulation is an injectable solution.

In another preferred embodiment, the pharmaceutical formulation further contains additional active ingredients.

Preferably, the additional active ingredients include BMP4, CCL28, anti-CD8 antibodies, or a combination thereof.

In the seventh aspect of the present invention, it provides a use of the active ingredient of the third aspect of the present invention, or the expression vector of the fourth aspect of the present invention, or the host cell of the fifth aspect of the present invention, or the pharmaceutical formulation of the sixth aspect of the present invention, in the preparation of a drug for treating heart diseases associated with cardiac injury and/or diseases associated with lymphatic abnormalities.

In another preferred embodiment, heart diseases associated with cardiac injury are selected from the group consisting of: myocardial infarction, myocarditis, idiopathic dilated cardiomyopathy, Chagas' cardiomyopathy, rheumatic heart disease, heart damage caused by coronavirus disease 2019 (COVID-19), a combination thereof.

In another preferred embodiment, diseases associated with lymphatic abnormalities are selected from the group consisting of: primary lymphedema caused by lymphatic malformation, secondary lymphedema caused by lymphatic rupture or obstruction, Turner syndrome, yellow nail syndrome, Hennekam syndrome, and a combination thereof.

In the eighth aspect of the present invention, it provides a method for screening promoting agents of TBX1 protein, comprising the steps of: administering a candidate drug to lymphatic endothelial cells cultured *in vitro* and/or experimental animals; if the TBX1 mRNA level or TBX1 protein level in the lymphatic endothelial cells cultured *in vitro* or the lymphatic vessels of the experimental animals is higher relative to the control group that did not receive the candidate drug, or if the function of TBX1 protein is enhanced relative to the control group that did not receive the candidate drug, then the candidate drug can be considered a promoting agent of TBX1 protein.

In another preferred embodiment, the promoting agent of TBX1 protein can be used to treat heart diseases associated with cardiac injury.

In another preferred embodiment, the promoting agent of TBX1 protein can be used to treat diseases associated with lymphatic abnormalities.

In the ninth aspect of the present invention, it provides a method for treating heart diseases associated with cardiac injury, comprising the steps of: administering the active ingredient of the third aspect of the present invention or the pharmaceutical formulation of the sixth aspect of the present invention to a subject in need thereof.

In another preferred embodiment, the subject in need thereof has heart diseases associated with cardiac injury.

In another preferred embodiment, heart diseases associated with cardiac injury are selected from the group consisting of: myocardial infarction, myocarditis, idiopathic dilated cardiomyopathy, Chagas' cardiomyopathy, rheumatic heart disease, heart damage caused by coronavirus disease 2019 (COVID-19), and a combination thereof.

In the tenth aspect of the present invention, it provides a method for treating diseases associated with lymphatic abnormalities, comprising the steps of: administering the active ingredient of the third aspect of the present invention or the pharmaceutical formulation of the sixth aspect of the present invention to a subject in need thereof.

In another preferred embodiment, the subject in need thereof has diseases associated with lymphatic abnormalities.

In another preferred embodiment, diseases associated with lymphatic abnormalities are selected from the group consisting of: primary lymphedema caused by lymphatic malformation, secondary lymphedema caused by lymphatic rupture or obstruction, Turner syndrome, yellow nail syndrome, Hennekam syndrome, and a combination thereof.

It should be understood that within the scope of the present invention, the various technical features described above and those specifically described in the following examples can be combined with each other to form new or preferred technical solutions. Due to limitations in space, these combinations are not exhaustively listed here.

## DESCRIPTION OF THE DRAWINGS

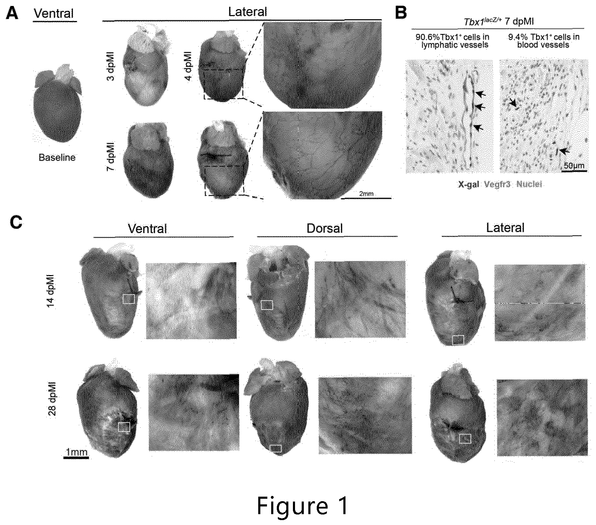

Figure 1 illustrates the activation of *Tbx1* expression in lymphatic endothelial cells after myocardial infarction (MI). (A) Expression of *Tbx1* in the heart at 3 or 7 days post-MI (dpMI). Sham heart (baseline, n=2), 3 dpMI (n=2), 4 dpMI (n=2), and 7 dpMI (n=6). (B) X-gal stained heart sections at 7 dpMI with Vegfr3 immunostaining (n=3). Tbx1 is mainly expressed in lymphatic endothelial cells. (C) Whole-heart X-gal staining at 14 dpMI (n=3) and 28 dpMI (n=3). dpMI: days post-MI.

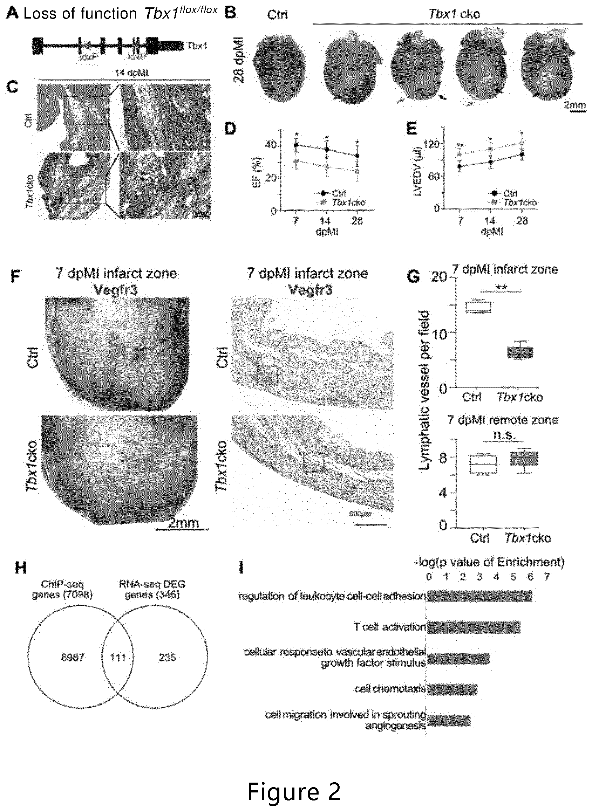

Figure 2 illustrates that endothelial cell-specific knockout of *Tbx1* (Tbx1cko) results in abnormal neo-lymphangiogenesis and impaired repair following MI. (A) Scheme of generating functional *Tbx1* knockout mice, where the functional gene sequences between red arrowheads are deleted upon Cre expression. (B) Ventral view of hearts at 28 dpMI. Black arrows, anterior ventricular wall aneurysm. Green arrows, postoperative adhesion. (C) Heart sections stained with hematoxylin and eosin at 14 dpMI (n=3). Green arrows, mononuclear infiltrating inflammatory cells. Blue arrows, hemorrhage spots. (D) Changes in ejection fraction (EF) after MI in control and *Tbx1cko* groups (n=7). Data are presented as mean $\pm$ standard error. *, p<0.05. Mann-Whitney U test. (E) Changes in left ventricular end-diastolic volume (LVEDV) after MI in control and Tbx1cko groups (n=7). Data are presented as mean $\pm$ standard error. *, p<0.05. **, p<0.01. Mann-Whitney U test. (F, G) Vegfr3 staining showed the status of neo-lymphangiogenesis at the infarct site of 7 dpMI hearts, including wholemount views of hearts and microscopic views of heart sections (F) and quantification of lymphatic endothelial cells in the infarct area and remote area (G). **, p<0.01. Mann-Whitney U test. (H) Differential gene expression of endothelial-specific transcription factors and downstream target genes analyzed by chromatin immunoprecipitation combined with high-throughput sequencing (ChIP-seq) to identify Tbx1 downstream targets. (I) Functional annotation of Tbx1 target genes.

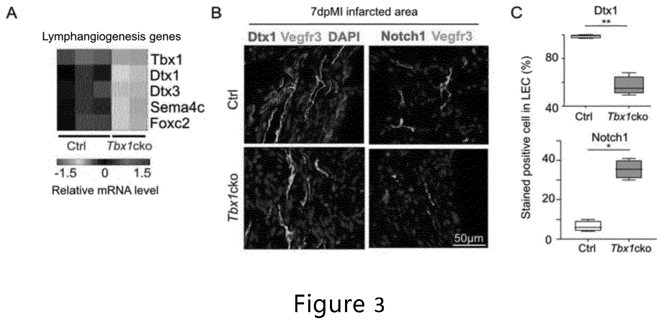

Figure 3 illustrates significant changes in the expression of lymphangiogenic genes in *Tbx1*-deficient hearts. (A) Transcriptome analysis indicated significant downregulation of lymphangiogenic genes Dtx1, Dtx3, Sema4c, and Foxc2 in cardiac endothelial cells after MI. (B, C) Immunofluorescence staining of Dtx1 and Notch1 in the infarcted area of 7 dpMI hearts showing the distribution and quantification of Dtx1- and Notch1-positive lymphatic endothelial cells.

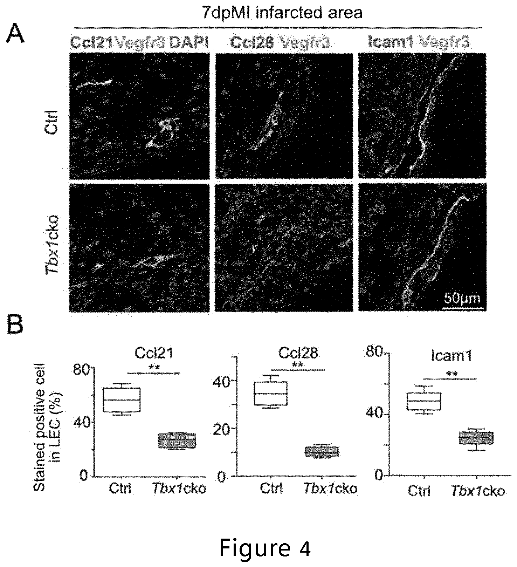

Figure 4 demonstrates significant changes in the expression of immunomodulatory genes in lymphatic endothelial cells of Tbx1cko hearts. (A, B) Immunofluorescence staining of Ccl21, Ccl28, and Icam1 in the infarcted area of 7 dpMI hearts showing the distribution and quantification of Ccl21-, Ccl28- and Icam1-positive lymphatic endothelial cells.

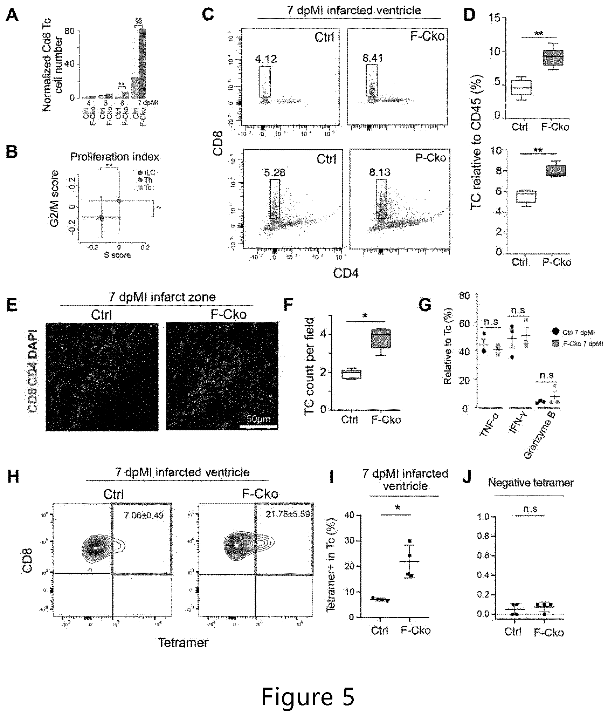

Figure 5 illustrates a significant increase in autoreactive CD8$^+$ T cells in *Tbx1*-deficient hearts. (A) Distribution of CD8$^+$ T cell numbers in single-cell RNA sequencing results, **, p = $3 \times 10^{-3}$. §§, p = $5 \times 10^{-15}$. Chi-square test. (B) Analysis of T cell subset cell proliferation revealed highly proliferative CD8$^+$ T cells. Data are presented as mean $\pm$ standard deviation, **, p<$5.6 \times 10^{-14}$. §§, p<$4.3 \times 10^{-11}$. Student's *t*-test. (C, D) Flow cytometry analysis of changes in the number of CD8$^+$ T cells in the hearts of endothelial-specific knockout (F-Cko) and lymphatic endothelial-specific knockout (P-Cko) mice at 7 dpMI. Data are presented as mean $\pm$ standard error (n=5). *, p<0.05. **, p<0.01. Mann-Whitney U test. (E, F) Immunofluorescence staining and quantification of CD8$^+$ T cells in the infarcted area of 7 dpMI hearts. Data are presented as mean $\pm$ standard error (n=5), *, p<0.05. Mann-Whitney U test. (G) Flow cytometry analysis of secreted cytotoxic factors in CD8$^+$ T cells. Data are presented as mean $\pm$ standard error (n=3). n.s., no significant difference. Mann-Whitney U test. (H, I) Flow cytometry analysis of Myh6 peptide-MHC I tetramer staining. F-Cko group shows a significant increase in autoreactive CD8$^+$ T cells at 7 dpMI hearts. Data are presented as mean $\pm$ standard error (n=4). *, p<0.05. Mann-Whitney U test. (J) Flow cytometry analysis of negative control peptide-MHC I tetramer staining on CD8$^+$ T cells from post-MI hearts. Data are presented as mean $\pm$ standard

error (n=4). n.s., no significant difference. Mann-Whitney U test.

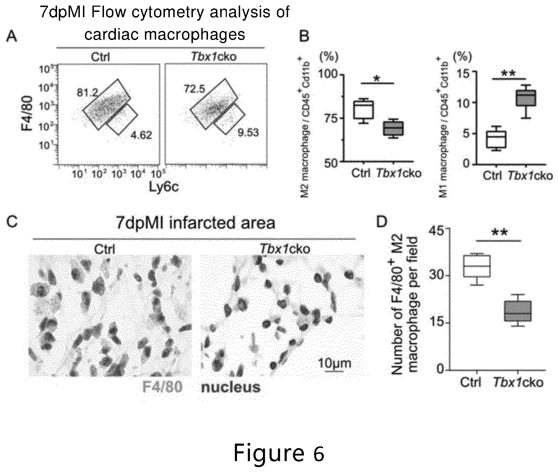

Figure 6 illustrates a decrease in proinflammatory M1 macrophages and a significant increase in reparative M2 macrophages in *Tbx1*-deficient hearts. (A, B) Flow cytometry analysis of changes in macrophage numbers in *Tbx1Cko* hearts at 7 dpMI. *, p<0.05. **, p<0.01. Mann-Whitney U test. (C, D) Immunohistochemical staining of M2 macrophages in the infarcted area of *Tbx1Cko* hearts at 7 dpMI. **, p<0.01. Mann-Whitney U test.

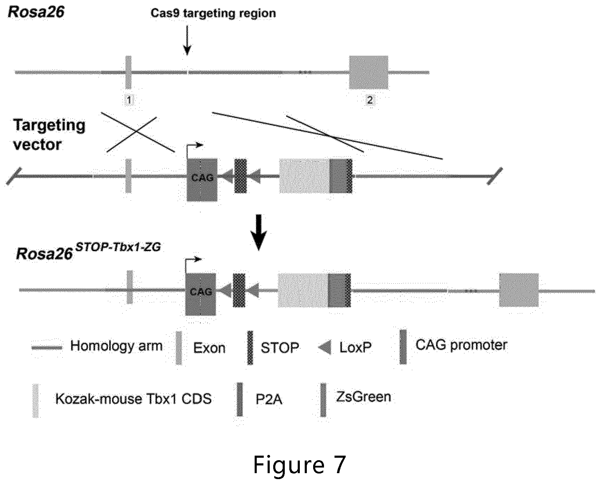

Figure 7 illustrates the schematic diagram of the mouse *Rosa26$^{STOP-Tbx1-ZG}$* allele construction.

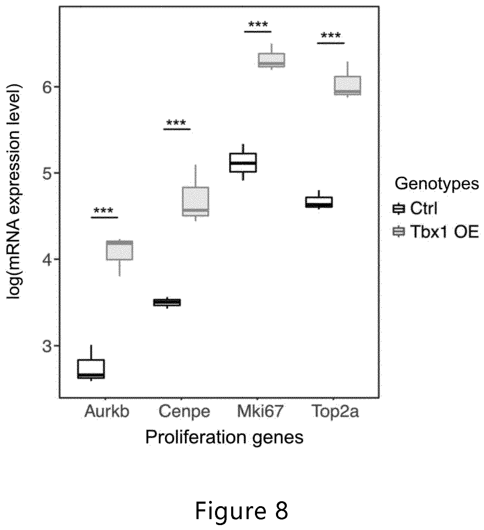

Figure 8 illustrates the significant increase in mRNA expression levels of cell proliferation-related genes in cardiac endothelial cells of *Tbx1* overexpression mice (*Tbx1*OE) compared to the control group. ***, $p<1\times10^{-3}$.

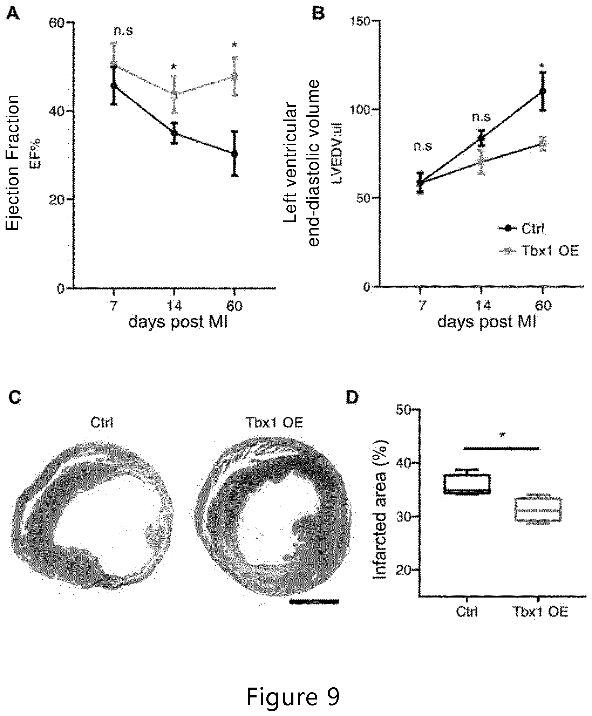

Figure 9 illustrates that overexpression of *Tbx1* in cardiac lymphatic endothelial cells improves myocardial repair after MI. (A, B) Echocardiograph measurement of ejection fraction (EF, A) and left ventricular end-diastolic volume (LVEDV, B) at 7 dpMI in control (n=8) and *Tbx1* OE groups (n=10). Mann-Whitney U test. n.s., no statistical difference. *, p < 0.05. (C) Masson's trichrome staining revealed the scar tissue in the cross section of 60 dpMI heart (scar stained in blue). (D) The infarcted zone in the *Tbx1*OE group (n=5) was significantly smaller than that of the control group (n=4). Mann-Whitney U-test. *, p < 0.05.

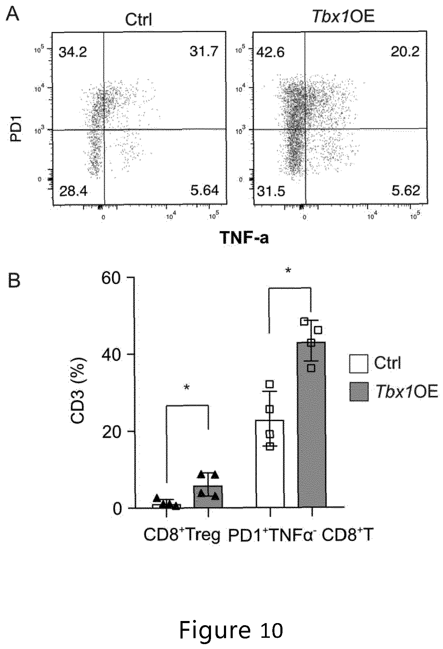

Figure 10 illustrates that lymphatic endothelial overexpression of *Tbx1* reduces cytotoxic CD8$^+$ T cells in hearts. (A, B) Flow cytometry analysis of 7 dpMI *Tbx1* OE hearts indicated changes in the number of Cd8$^+$ T cells. Data are shown as mean $\pm$ standard error (n=5). *, p<0.05. Mann-Whitney U test. The number of PD-1$^+$TNF$\alpha^-$ exhaustive CD8$^+$ T cells significantly increased in *Tbx1*OE hearts. The number of Foxp3$^+$CD25$^+$ regulatory CD8$^+$ T cells (CD8$^+$Treg) significantly increased in *Tbx1*OE hearts.

## DETAILED DESCRIPTION

Through extensive and in-depth research, the inventors have unexpectedly developed an active ingredient for the treatment of heart diseases related to cardiac injury for the first time. The inventors discovered that overexpression of the TBX1 protein in cardiac lymphatic endothelial cells promotes lymphatic endothelial cell proliferation. More important, TBX1 plays an immunomodulatory role in promoting the establishment of an immunosuppressive microenvironment in the post-myocardial infarction (MI) heart, thereby inhibiting autoimmune reactions after cardiac injury, alleviating concurrent cardiac tissue inflammation, and facilitating the repair and regeneration of damaged cardiac tissue. Experimental evidence demonstrates that overexpressing TBX1 in cardiac lymphatic endothelial cells significantly improves cardiac function after MI and promotes the repair of post-MI cardiac tissue. Based on these findings, the present invention has been completed.

### Terms

To facilitate a better understanding of this disclosure, certain terms are first defined. As used herein, unless otherwise explicitly stated, each term below should have the meaning described below. Other definitions are provided throughout the specification.

The term "approximately" can refer to a value or composition within an acceptable range of error determined by ordinarily skilled technicians in this field, which partly depends on how the value or composition is measured or determined. For example, as used in this specification, "approximately 100" includes all values between 99 and 101 (e.g., 99.1, 99.2, 99.3, 99.4, etc.).

As used in this specification, the terms "comprising" or "including (containing)" can be open, semi-closed, or closed. In other words, the terms also encompass "essentially consisting of" or "consisting of."

As used in this specification, the terms "subject" and "individual in need" refer to any mammal or non-mammal. Mammals include, but are not limited to, humans, vertebrates such as rodents, non-human primates, cattle, horses, dogs, cats, pigs, sheep, and goats.

### TBX1

The present invention provides an active ingredient for repairing cardiac injury, the active ingredient comprising: the TBX1 protein, its encoding sequence, its enhancers, or a combination thereof.

The TBX1 gene encodes a T-box transcription factor and is located in the human chromosome 22q11.2 region. It is the major disease gene of 22q11.2 microdeletion syndrome. The disease has a prevalence of up to 1 :2000 in newborns. 75% of affected children have congenital heart disease, accompanied by craniofacial developmental abnormalities, thymic aplasia, and other congenital defects. In early embryonic development, Tbx1 is expressed in a subset of differentiating cardiac progenitor cells and is also crucial for the development of the cardiac lymphatic system. However, the expression level of Tbx1 in the heart is extremely low in adulthood.

The gene ID of the TBX1 gene in the NCBI database is 6899, and its expressed mRNA sequences have four isoforms: NM_005992.1, NM_080646.2, NM_080647.1, and NM_001379200.1. The mouse Tbx1 gene in the NCBI database has a gene ID of 21380, and its expressed mRNA sequences have four isoforms: NM_011532.2, NM_001285472.1, NM_001285476.1, and NM_001373938.1. As used herein, the encoding sequence of the TBX1 protein can be any of the above sequences or their variants, as long as the sequence can effectively express a functionally normal or enhanced TBX1 protein. The function is to promote lymphangiogenesis and the formation of an immunosuppressive microenvironment.

In another preferred embodiment, the encoding sequence of the TBX1 protein is as shown in SEQ ID NO. 1.

ATGCACTTCAGCACCGTCACCAGGGACATGGAAGCCTTCACGGCCAGCAGCCTGAGC
AGCCTGGGGGCCGCGGGGGGCTTCCCGGGCGCCGCGTCGCCCGGCGCCGACCCGTA
CGGCCCGCGCGAGCCCCGCCGCCGCCGCCGCGCTACGACCCGTGCGCCGCCGCCG
CCCCCGGCGCCCCGGGCCCGCCGCCGCCGCCGCACGCCTACCCGTTTGCGCCGGCCG
CCGGGGCCGCCACCAGCGCCGCCGCCGAGCCCGAGGGCCCCGGGGCCAGCTGCGCG
GCCGCAGCCAAGGCGCCGGTGAAGAAGAACGCGAAGGTGGCCGGTGTGAGCGTGC
AGCTAGAGATGAAGGCGCTGTGGGACGAGTTCAACCAGCTGGGCACCGAGATGATC
GTCACCAAGGCCGGCAGGCGGATGTTTCCCACCTTCCAAGTGAAGCTCTTCGGCATG
GATCCCATGGCCGACTATATGCTGCTCATGGACTTCGTGCCGGTGGACGATAAGCGCT
ACCGGTACGCCTTCCACAGCTCCTCCTGGCTGGTGGCGGGGAAGGCCGACCCTGCCA
CGCCAGGCCGCGTGCACTACCACCCGGACTCGCCTGCCAAGGGCGCGCAGTGGATG
AAGCAAATCGTGTCCTTCGACAAGCTCAAGCTGACCAACAACCTACTGGACGACAA
CGGCCACATTATTCTGAATTCCATGCACAGATACCAGCCCCGCTTCCACGTGGTCTATG
TGGACCCACGCAAAGATAGCGAGAAATATGCCGAGGAGAACTTCAAAACCTTTGTGT
TCGAGGAGACACGATTCACCGCGGTCACTGCCTACCAGAACCATCGGATCACGCAGC
TCAAGATTGCCAGCAATCCCTTCGCGAAAGGCTTCCGGGACTGTGACCCTGAGGACT
GGCCCCGGAACCACCGGCCCGGCGCACTGCCGCTCATGAGCGCCTTCGCGCGCTCGC
GGAACCCCGTGGCTTCCCCGACGCAGCCCAGCGGCACGGAGAAAGACGCGGCTGAG
GCCCGGCGAGAATTCCAGCGCGACGCGGGCGGGCCAGCAGTGCTCGGGGACCCGGC

GCATCCTCCGCAGCTGCTGGCCCGGGTGCTAAGCCCCTCGCTGCCCGGGGCCGGCGG
CGCCGGCGGCTTAGTCCCGCTGCCCGGCGCGCCCGGAGGCCGGCCCAGTCCCCCGA
ACCCCGAGCTGCGCCTGGAGGCGCCCGGCGCATCGGAGCCGCTGCACCACCACCCCT
ACAAATATCCGGCCGCCGCCTACGACCACTATCTCGGGGCCAAGAGCCGGCCGGCGC
CCTACCCGCTGCCCGGCCTGCGTGGCCACGGCTACCACCCGCACGCGCATCCGCACC
ACCACCACCACCCCGTGAGTCCAGCCGCCGCGGCCGCCGCCGCCGCTGCCGCAGCT
GCCGCGGCCGCCAACATGTACTCGTCGGCCGGAGCCGCGCCGCCCGGCTCCTACGAC
TATTGCCCCAGATAA(SEQ ID NO: 1).

In another preferred embodiment, the encoding sequence of the Tbx1 protein is as the CDS sequence used in the examples:

ATGATCTCCGCCGTGTCTAGTCCGTGGCTCACGCAGCTCTCGCACTTCTGCGACGTTG
CAGCCTTCGCAGCCAGCAGTCTGAGCGGCCTGGGATCCCCGTCGCCTGGCGCCGACC
CGTTCGGCCCTCGCGAGCCGCCGCCACCGCGCTACGATCCGTGCGCTGCAGTCCCCG
GTGCCCCGGGCCGCCGCCGCGCGCCTATCCTTTCGCGCCCGCCCCCGGGGCGG
CTGGCAGCTCGGCGGCGGAGTCCGAGGGTCCGGGGGCTAGCCGCGCGGCTGCGGTC
AAGGCTCCGGTGAAGAAGAACCCGAAGGTGGCCAGCGTGAGCGTGCAGCTGGAGAT
GAAGGCGCTGTGGGACGAGTTCAATCAGCTGGGCACCGAGATGATCGTCACCAAGG
CAGGCAGACGAATGTTCCCCACGTTCCAAGTGAAGCTTTTGGAATGGATCCCATGG
CCGACTACATGCTGCTCATGGACTTTGTGCCCGTAGATGACAAGCGCTACCGGTATGC
TTTCCATAGCTCCTCCTGGCTGGTGGCCGGCAAGGCAGATCCTGCTACACCTGGCCGA
GTACACTACCACCCGGACTCGCCGGCTAAGGGCGCACAGTGGATGAAACAGATTGTG
TCTTTCGACAAGCTGAAACTGACCAATAACCTGCTGGATGACAATGGCCATATTATTC
TCAACTCCATGCACAGATATCAGCCCCGATTCCATGTTGTCTATGTGGACCCTCGAAA
AGACAGTGAGAAATATGCAGAGGAGAACTTCAAAACTTTTGTGTTTGAGGAGACAC
GCTTCACTGCAGTCACTGCCTACCAGAATCACCGGATCACGCAGCTTAAGATTGCCA
GCAACCCCTTCGCCAAAGGCTTCCGGGATTGCGACCCGGAGGACTGGCCCCGGAAC
CACCGGCCCGGAGCGCTGCCGCTCGTGAGTGCCTTTGCTCGCTCTCGGAATCCCGTG
GCTTCCCCCACGCAGCCCAATGGCTCAGACAAAGACGCTGCAGAAGCCCGGCGCGA
GTTCGACCGTGACTCCGGACCCGCAGCGCTCGGCGACGCTACGCACCCGCCGCAGCT
GCTGGCGCGCGTGCTGAGCCCCGCACTGCCCGGGCCTGGCGGCCTCGTCCCGCTACC
CGGCGGATCCGGAGGCCGCCACAGTCCCCGCACGCCGATCTGCGCCTGGAGGCGC
CGGGCGCGTCCGAGCCGCTGCACCACCATCCCTACAAGTACCCGGCCGCCGCCTACG
ACCACTACCTCGGGGCCAAGAGCCGGCCGGCGCCCTACCCGCTGCCAGGCCTGCGC
GGCCACGGCTACCACCCGCACGCGCACCCGCACGCGCACCCGCACCATCACCACCA
CCCCGCGGTGAACCCGGCCGCCGCCGCCGCTGCTGCCGCAGCAGCCAACGTGTACTC
GTCGGCGGCCGCGCCGCCCGGTGCCTACGACTACTGCCCCAGATAG(SEQ ID NO: 2).

**Cardiac injury**

As used herein, the term "cardiac injury" refers to damage to cardiac tissue that includes degeneration and necrosis of myocardial cells, as well as degeneration and necrosis of cardiac blood vessels and lymphatic vessels. Following cardiac injury, inflammation and autoimmune reactions occur.

The present invention has discovered that TBX1 protein, TBX1 mRNA, or its promoting agents can be used to repair cardiac injuries. Overexpression of TBX1 in cardiac lymphatic endothelial cells not only promotes lymphatic endothelial cell proliferation but also regulates autoimmune reactions, thereby alleviating cardiac inflammation. Thus, these can be employed to treat heart diseases associated with cardiac injury.

The term "heart diseases associated with cardiac injury" encompasses ischemia, acute myocardial injury caused by hypoxia, autoimmune cardiac diseases, and other diseases leading to myocardial damage. In another preferred example, heart diseases associated with cardiac injury are selected from the group consisting of: myocardial infarction, myocarditis, idiopathic dilated cardiomyopathy, Chagas' cardiomyopathy, rheumatic heart disease, heart damage caused by coronavirus disease 2019 (COVID-19), and a combination thereof.

**Expression vectors and host cells**

The present invention provides an expression vector for expressing the TBX1 protein, which contains the encoding sequence of the TBX1 protein of the present invention.

With the provided sequence information, skilled technicians can use available cloning techniques to generate nucleic acid sequences or vectors suitable for transfection/transduction into cells.

In another preferred embodiment, the expression vector is selected from the group consisting of: DNA, RNA, plasmids, eukaryotic expression vectors, prokaryotic expression vectors, lentiviral vectors, adenoviral vectors, adeno-associated viral vectors, retroviral vectors, transposons, and a combination thereof.

Preferably, a nucleic acid sequence encoding the TBX1 protein is included in the vector, preferably provided as an expression vector. It is preferably provided as a gene therapy vector that is optimally suitable for transfection/transduction and expression in target cells (e.g., cochlear supporting cells). The vector can be viral (e.g., adenovirus) or non-viral (e.g., plasmid). Viral vectors include those derived from adenoviruses, including mutated forms of adeno-associated viruses (AAV), retroviruses, lentiviruses, herpesviruses, vaccinia viruses, bovine poxviruses, MMLV, GaLV, simian immunodeficiency viruses (SIV), HIV, poxviruses, and SV40. Preferably, the viral vector is replication-defective or replication-deficient, capable of replicating or conditionally replicating. The viral vector can typically maintain an extrachromosomal state without integrating into the target cell genome. An AAV vector is a preferred viral vector for introducing nucleic acid sequences encoding the TBX1 protein into target cells. The use of specific AAV serotypes (AAV serotypes 2 to AAV serotype 12) or modified versions of these serotypes can achieve selective targeting.

Viral vectors possess the ability to enter cells. However, non-viral vectors such as plasmids can be complexed with reagents to facilitate uptake by target cells. Such reagents include cationic polymers. Optionally, delivery systems such as lipid-based delivery systems can be used. The vectors used in the present invention are preferably suitable for in vivo or ex vivo use and are preferably suitable for use in humans.

Vectors will preferably include one or more regulatory sequences to guide the expression of nucleic acid sequences in target cells. Regulatory sequences can include promoters, enhancers, transcription termination signals, polyadenylation sequences, replication origins, nucleic acid restriction sites, and homologous recombination sites associated with nucleic acid sequences operably linked to them. Vectors may also include selective markers, for example, to determine the expression of vectors in growth systems (e.g., bacterial cells) or target cells.

"Operably linked" means that the nucleic acid sequence is functionally associated with the sequence it is operably linked to, such that they are connected in a manner that affects their expression or function in relation to each other. For example, a nucleic acid sequence operably linked to a promoter will have an expression pattern influenced by the promoter.

The present invention also provides a host cell for expressing the TBX1 protein. Host cells can be prokaryotic cells such as bacterial cells, or lower eukaryotic cells such as yeast cells, or higher eukaryotic cells such as mammalian cells. Representative examples include Escherichia coli, Bacillus subtilis; bacterial cells of Salmonella typhimurium; fungal cells such as yeast; insect cells such as Drosophila S2 or Sf9; and animal cells such as CHO, COS7, 293 cells, T cells, and NK cells.

**Pharmaceutical formulation**

The present invention provides a pharmaceutical formulation or composition comprising: (a) the active ingredient as described in the second aspect of the present invention, or the vector as described in the third aspect of the present invention, or the host cell as described in the fourth aspect of the present invention, and (b) a pharmaceutically acceptable carrier, excipient, or diluent.

In another preferred embodiment, the pharmaceutical formulation is used for repairing heart tissue injuries.

In another preferred embodiment, the pharmaceutical formulation is used for treating heart diseases associated with cardiac injury.

In another preferred embodiment, the pharmaceutical formulation is used for treating diseases associated with lymphatic abnormalities.

The "active ingredient" in the pharmaceutical formulation of the present invention refers to TBX1 protein, its encoding sequence, or its promoting agent, or a combination thereof, or the expression vector of the present invention, such as a viral vector. The "active ingredient", formulation, and/or composition of the present invention can be used to treat heart diseases associated with cardiac injury and/or diseases associated with lymphatic vessel abnormalities. The term "safe and effective dose" refers to an amount of the active ingredient sufficient to significantly improve the condition or symptoms without causing severe side effects. "Pharmaceutically acceptable carrier or excipient" refers to one or more compatible solid or liquid fillers or gel substances that are suitable for human use and must have sufficient purity and sufficiently low toxicity. "Compatibility" here refers to the ability of the components in the composition to mix with each other and with the active ingredient of the present invention without significantly reducing the efficacy of the active ingredient.

The formulations and/or compositions of the present invention may also include BMP4, CCL28, anti-CD8 antibodies, and other active ingredients similar in function to the TBX1 molecule. The formulations and/or compositions can be used in combination with other known drugs for treating heart diseases associated with cardiac injury and/or diseases associated with lymphatic abnormalities in the field.

The composition can be in liquid or solid form, such as powder, gel, or paste. Preferably, the composition is in liquid form, preferably injectable liquid. Suitable excipients will be known to those skilled in the field.

In any administration mode, preferably, the carrier is provided as an injectable liquid. Preferably, the injectable liquid is provided as capsules or syringes.

Examples of pharmaceutically acceptable carriers include cellulose and its derivatives (such as sodium carboxymethyl cellulose, ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agents (such as sodium dodecyl sulfate), colorants, flavorings, stabilizers, antioxidants, preservatives, and deionized water.

The composition can include physiologically acceptable sterile aqueous or non-aqueous solutions, dispersions, suspensions, or emulsions, and sterile powders for reconstitution into sterile injectable or dispersible solutions. Suitable aqueous and non-aqueous carriers, diluents, solvents, or excipients include water, ethanol, polyols, and suitable mixtures thereof.

**Method for Screening promoting agents of TBX1 Protein:**

The present invention also provides a method for screening promoting agents of the TBX1 protein, comprising the steps of: administering candidate drugs to *in vitro* cultured lymphatic endothelial cells and/or experimental animals. If the TBX1 mRNA level, TBX1 protein level, or TBX1 protein function in the *in vitro* cultured lymphatic endothelial cells or lymphatic vessels in experimental animals is elevated relative to the control group that did not receive the candidate drug, then the candidate drug can be considered a promoting agent of the TBX1 protein.

In the present invention, the term "candidate drug" includes, but is not limited to: small molecule compounds, nucleic acid drugs, or protein drugs. The candidate drug may directly or indirectly affect TBX1, but ultimately leads to an increase in TBX1 protein function, level, or TBX1 mRNA level in *in vitro* cultured lymphatic endothelial cells or lymphatic vessels in experimental animals.

In another preferred example, the promoting agent of the TBX1 protein can be used for treating heart diseases associated with cardiac injuries.

The main advantages of the present invention are as follows:

(1) After cardiac injuries, autoimmune damage tends to occur, but currently, there are no effective means to control this pathological mechanism, resulting in the occurrence of heart failure in a significant number of patients after cardiac injuries. The present invention proposes a means to control this pathological mechanism, which may provide new therapeutic methods for heart diseases caused by this mechanism.

(2) The present invention introduces a novel endogenous cardiac repair and regeneration mechanism. The active ingredient (or combination) of the present invention enhances post-injury repair and regeneration of the heart, from two different levels, by inhibiting autoimmune reactions and promoting lymphatic endothelial cell proliferation, thereby achieving a good effect. This provides a new strategy and approach for human post-myocardial infarction repair and regeneration.

(3) Additionally, the favorable microenvironment created by the active ingredient (or combination) of the present invention through inhibiting autoimmune reactions also offers permissive conditions for cell transplantation during myocardial infarction repair and regeneration.

Next, specific examples are provided to further illustrate the present invention. It should be understood that these examples are intended for illustrative purposes only and are not intended to limit the scope of the present invention. Experimental methods without specific conditions in the following examples are generally carried out under conventional conditions as described in references like "Molecular Cloning: A Laboratory Manual" by Sambrook et al., Cold Spring Harbor Laboratory Press, 1989, or according to the conditions recommended by manufacturers. Unless otherwise indicated, percentages and ratios are calculated by weight.

Materials, reagents, instruments, etc., used in the examples, if not specifically mentioned, can be obtained through commercial sources.

**Experimental Methods:**

(1) Generation of *Rosa26-STOP-Tbx1/STOP-Tbx1*(Tbx1OE) Mouse Strain: The LoxP-STOP-LoxP-Tbx1 cDNA-P2A-ZsGreen cassette was inserted into the Rosa26 gene locus, where Tbx1 cDNA consists of the nucleotide sequence encoding the TBX1 protein's open reading frame, as shown in SEQ ID NO: 2 (Figure 7). After Cre-mediated recombination at the LoxP sites, the STOP cassette is excised, allowing Tbx1 expression. The *Rosa26-STOP-Tbx1/STOP-Tbx1* (Tbx1OE) mice may live in a healthy condition.

(2) Transcriptional Sequencing and Analysis: RNA was extracted using TRIzol®LS reagent (ThermoFisher science), and high-throughput sequencing was conducted using 1 μg of total RNA with Illumina platform library preparation methods. Each sample generated at least 15 million paired-end reads. The STAR software (version 2.5.2b, MIT license) was used to align the raw sequences with the mouse genome (Gencode, GRCm38, Open Access). Gene expression levels were statistically analyzed on a per-gene basis. Differential expression analysis was performed using the DESeq2 package (version 1.20.0, LGPL license ≥ 3) in R language (criteria for differential expression: fold change ≥ 1.5 and p-value ≤ 0.05). Subsequently, the differential genes were functionally analyzed using Metascape (https://metascape.org/gp/index.html#/menu/term_service).

(3) Myocardial Infarction Animal Model: An 8-12 week-old mouse model of myocardial infarction was employed, following previously described methods with subtle modifications. Briefly, the experimental steps were as follows: After intubation of the mouse's trachea, connect it to a ventilator (Harvard apparatus). Anesthesia was maintained with 3%-5% isoflurane, ventilation frequency of 100 breaths/minute, and tidal volume of 350 μl. Ligature of the left anterior descending coronary artery induced myocardial infarction. The sham-operated group underwent the same surgical steps except for coronary artery ligation. After the animals regained consciousness, subcutaneous injections of buprenorphine (0.1 mg/kg) were administered, with a second injection 24 hours later.

(4) Measurement of Cardiac Function: Echocardiography was conducted through the thorax of mice subjected to left anterior descending coronary artery ligation for 7, 14, and 28 days. The Vevo 2100 system (Visual Sonics) was utilized, with a probe frequency of 30 MHz and a frame rate of 30 frames/second. Measurements were taken from the cross-section of long-axis images of the left ventricular cardiac cycle, and the following formulas were employed to calculate left ventricular end-systolic volume (LVESV), end-diastolic volume (LVEDV), and left ventricular ejection fraction (LVEF).

$$LV \ volume = \frac{8\pi}{3} \times \frac{endocardial \ area^2}{endocardial \ long \ axis \ length}$$

$$LVEF = \frac{LVEDV - LVESV}{LVEDV} \times 100$$

**Example 1**

**Activation of Tbx1 Expression in Cardiac Lymphatic Endothelial Cells after Myocardial Infarction**

Following induction of myocardial infarction in adult mice, the expression of Tbx1 in cardiac endothelial cells continued to increase, and 90% of Tbx1-positive cells were identified as lymphatic endothelial cells (Figures 1A-1C).

**Example 2**

**Impaired Recovery After Myocardial Infarction Due to Lymphatic Endothelial-Specific Deletion of Tbx1**

A mouse model with tissue-specific deletion of Tbx1 in cardiac lymphatic endothelial cells was generated (Figure 2A). Myocardial infarction was induced in both control and knockout mice. Compared to the control group, Tbx1-deficient mice exhibited worsened recovery after myocardial infarction, characterized by severe tissue adhesion and formation of ventricular aneurysms (Figure 2B). Histological examination indicated delayed inflammation regression (Figure 2C). Decreased cardiac ejection function and increased left ventricular end-diastolic volume were observed (Figures 2D-2E). Cellular analysis revealed significantly impaired cardiac lymphangiogenesis in Tbx1-deficient mice (Figures 2F-2G).

Transcriptome sequencing (Figure 3A) and immunofluorescence staining (Figure 3B) demonstrated altered expression of genes associated with lymphatic vessel growth upon Tbx1 deletion: the expression of genes promoting lymphatic endothelial cell proliferation, such as Dtx1, Dtx3, Sema4c, and Foxc2, was downregulated in cardiac endothelial cells one week after myocardial infarction. Meanwhile, the expression of the Notch1 gene, which inhibits lymphatic vessel growth, increased (Figure 3C).

**Example 3**

**Tbx1 Regulates Autoimmune Response After Myocardial Infarction**

Chromatin immunoprecipitation sequencing (ChIP-seq) detected binding sites of the transcription factor Tbx1 on the cardiac endothelial cell genome. Genes related to autoimmune tolerance were significantly enriched, indicating Tbx1's potential role in regulating autoimmune response after myocardial infarction (Figures 2H-2I).

Endothelial-specific transcriptome analysis revealed that Tbx1 deficiency led to decreased mRNA levels of immunomodulatory genes, such as Icam1, Ccl21, and Ccl28, in endothelial cells. Icam1 is an adhesion protein involved in leukocyte migration and suppression of leukocyte immune activity. Ccl21 and Ccl28 are cytokines that regulate the immune microenvironment and attract immunosuppressive cells like regulatory T cells to inflammation sites, reducing autoimmune response. Immunofluorescence experiments showed reduced protein levels of Icam1, Ccl21, and Ccl28 in lymphatic endothelial cells of Tbx1-deficient mice seven days after myocardial infarction (Figures 4A-B).

Single-cell RNA sequencing analysis revealed active proliferation of CD8$^+$ T cells in the hearts of Tbx1-deficient mice, resulting in a significant increase in cell numbers (Figure 5A-5F), and similar cytotoxicity as CD8$^+$ T cells in the control group (Figure 5G). MHC I tetramer staining further indicated a significant increase in autoreactive CD8$^+$ T cells targeting $\alpha$-myosin heavy chain in the F-Cko group (Figures 5H-5J), suggesting elevated autoimmune response after Tbx1 knockout following myocardial infarction.

**Example 4**

**Tbx1 Deficiency Leads to Increased Macrophage Infiltration After Myocardial Infarction**

Myocardial infarction was induced in adult Tbx1-deficient mice, and macrophage subsets were analyzed by flow cytometry at the 7th day after myocardial infarction. Results revealed a significant decrease in M2 macrophages (characterized by high F4/80 expression and low Ly6c expression, promoting repair and inflammation resolution) and a significant increase in M1 macrophages (characterized by low F4/80 expression and high Ly6c expression, indicating inflammation and potential autoimmune activation) in the Tbx1-deficient group compared to controls (Figures 6A-B).

Immunohistochemistry staining also showed reduced M2 macrophage cell counts at the infarct site in the hearts of Tbx1-deficient mice at the 7th day after myocardial infarction (Figures 6C-D).

**Example 5**

**Overexpression of Tbx1 in Lymphatic Endothelial Cells Activates Lymphatic Endothelial Cell Proliferation**

Lymphatic endothelial cells play a critical role in myocardial infarction repair. During the transition from the acute inflammatory phase to the reparative phase, alterations in the transcriptome of lymphatic endothelial cells driven by Tbx1 not only promote cardiac lymphangiogenesis but also confer additional immunomodulatory functions. This helps suppress post-myocardial infarction autoimmune responses and prevent sustained attacks from autoreactive T cells and chronic inflammation. Therefore, in this example, we targeted lymphatic endothelial cells to investigate the feasibility of using Tbx1 overexpression to regulate the cardiac immune microenvironment for myocardial infarction treatment.

Firstly, *Rosa26$^{Stop-Tbx1-ZG}$* mice (Tbx1OE mice, Figure 7) were constructed. Lymphatic-specific Prox1Cre mice were crossed with Tbx1OE mice to obtain double heterozygous mice. Adult double heterozygous mice were orally administered tamoxifen to induce Tbx1 overexpression in lymphatic endothelial cells. Transcriptome sequencing was used to analyze changes in the gene expression profile of cardiac lymphatic endothelial cells. The molecular effects and potential target genes were investigated after enhancing Tbx1 expression.

Results revealed that activation of Tbx1 in cardiac lymphatic endothelial cells increased the expression of proliferation-related genes in lymphatic endothelial cells of adult mice hearts. Notably, expression levels of genes such as Top2a, Mki67, Cenpe, and Aurkb were more than doubled (n = 3, P < $1\times10^{-5}$) after Tbx1 overexpression (Figure 8).

**Example 6**

**Effects of Lymphatic Endothelial Tbx1 Overexpression on Post-Myocardial Infarction Recovery**

Lymphatic-specific Prox1Cre mice were crossed with Tbx1OE mice to obtain double heterozygous mice. Adult double heterozygous mice were orally administered tamoxifen to induce Tbx1 overexpression in lymphatic endothelial cells. One week later, these mice underwent coronary artery ligation to model myocardial infarction. Cardiac function was measured using echocardiography 7, 14, and 60 days after surgery.

Results indicated that Tbx1-overexpressing mice exhibited significantly improved cardiac ejection fraction two weeks after myocardial infarction (Figure 9A). This improvement was sustained for up to two months post-infarction, accompanied by a corresponding decrease in left ventricular end-diastolic volume (Figure 9B). Histological analysis revealed reduced scar tissue area in the hearts of Tbx1OE mice 60 days after myocardial infarction (Figure s9C-D).

## Example 7

## Lymphatic Endothelial Tbx1 Overexpression Reduces Cytotoxic CD8+ T Cells in the Heart

Lymphatic-specific Prox1Cre mice were crossed with Tbx1OE mice to obtain double heterozygous mice. Adult double heterozygous mice were orally administered tamoxifen to induce Tbx1 overexpression in lymphatic endothelial cells. One week later, these mice underwent coronary artery ligation to model myocardial infarction. Flow cytometry analysis was used to evaluate CD8+ T cell subsets at the 7th day after myocardial infarction.

Results indicated a significant increase in PD-1-expressing exhausted CD8+ T cells and a reduction in TNFα expression levels in CD8+ T cells in the Tbx1OE group (Figure 10A). Simultaneously, the number of immunosuppressive Foxp3+ CD25+ regulatory CD8+ T cells significantly increased in the Tbx1OE group (Figure 10B).

## Discussion

The occurrence of acute inflammation and injury repair following myocardial infarction is a complex, self-driven process involving various cell types such as immune cells, cardiomyocytes, vascular endothelial cells, and fibroblasts. Most research in the field of myocardial infarction repair and regeneration has focused on these cell types.

The results of the present invention suggest that lymphatic endothelial cells play a significant role in the repair of myocardial infarction damage. During the transition from the acute inflammatory phase to the repair phase, Tbxl-driven changes in the transcriptome of lymphatic endothelial cells not only promote lymphatic endothelial cell proliferation but also confer additional immunomodulatory functions. This helps suppress post-myocardial infarction autoimmune responses and prevent sustained attacks from self-reactive T cells and chronic inflammation. Unexpectedly, this invention revealed that Tbx1 in cardiac lymphatic endothelial cells regulates the intramyocardial microenvironment and coordinates post-myocardial infarction repair and regeneration. Tbx1 also assist in post-infarction repair through lymphatic endothelial cells.

Another distinctive feature of the present invention is the revelation of an immunomodulatory repair mechanism that starts at the begining of the post-myocardial infarction reparative stage. This mechanism is characterized by its unique stage and cell-type specificity, different from previous studies on immune intervention in myocardial infarction repair and regeneration. Prior research has shown that non-selective suppression of inflammation is detrimental to myocardial infarction repair. Current clinical studies of immune intervention in post-myocardial infarction treatment have not demonstrated clear therapeutic benefits. Therefore, this invention provides a new dimension to the orderly regulation mechanism of post-myocardial infarction repair and regeneration. By enhancing this novel endogenous repair and regeneration mechanism, new strategies and approaches to promote post-myocardial infarction recovery and repair are formed. Additionally, creating a favorable microenvironment through the inhibition of autoimmune reactions can also support cell transplantation for myocardial infarction repair and regeneration.

The heart is an organ prone to autoimmune responses. Certain cardiomyocyte-specific proteins are usually not expressed in the thymus, allowing autoreactive T cells against cardiomyocyte-specific antigens to escape central tolerance mechanisms and be released into the peripheral blood. Following cardiac injury, myocardial self-antigens are released, activate autoreactive T cells and lead to autoimmune reactions. Apart from acute myocardial injury caused by ischemia and hypoxia, various diseases leading to myocardial damage, such as myocarditis, certain idiopathic dilated cardiomyopathies, Chagas' disease, and rheumatic heart disease, are associated with persistent autoimmune reactions. Therefore, this invention's research suggests that overexpression of Tbx1 can also be used to treat other heart diseases related to autoimmune reactions, beyond myocardial infarction.

Furthermore, although the mechanisms presented in this invention are primarily based on a cardiac injury model, the genes regulated by Tbx1 and associated with lymphatic endothelial growth are generally expressed in lymphatic endothelial cells throughout various tissues. Thus, similar mechanisms can be applied to treat lymphatic abnormalities in various tissues, such as primary lymphedema caused by defective lymphatic development or secondary lymphedema resulting from lymphatic rupture or obstruction. It can also be applied to genetic syndromes characterized by lymphatic edema, such as Turner syndrome, hypoparathyroidism, yellow nail syndrome, and Hennekam syndrome.

All references mentioned in the present application are are incorporated by reference herein, as if each reference were cited separately. Furthermore, it should be understood that after reading the aforementioned disclosure of this invention, those skilled in the field can make various modifications or variations of the invention, all of which fall within the scope of the claims attached to the present application.

**Claims**

1.  Use of an active ingredient for preparing a formulation or drug, wherein the formulation or drug is used for:

    (i) activating proliferation of cardiac lymphatic endothelial cells;
    (ii) suppressing cardiac autoimmune reactions;
    (iii) promoting the transformation of M1-type macrophages to M2-type macrophages;
    (iv) repairing cardiac injury;
    (v) treating heart diseases associated with cardiac injury; and
    (vi) treating diseases associated with lymphatic abnormalities;

    wherein the active ingredient comprises: TBX1 protein, an encoding sequence thereof, or a promoting agent thereof, or a combination thereof.

2.  The use according to claim 1, wherein the heart diseases associated with cardiac injury are selected from the group consisting of: myocardial infarction, myocarditis, idiopathic dilated cardiomyopathy, Chagas' cardiomyopathy, rheumatic heart disease, heart damage caused by novel coronavirus disease (COVID-19), and a combination thereof.

3.  The use according to claim 1, wherein the diseases associated with lymphatic abnormalities are selected from the group consisting of: primary lymphedema caused by defective lymphatic development, secondary lymphedema caused by lymphatic rupture or obstruction, Turner syndrome, yellow nail syndrome, Hennekam syndrome, and other genetic syndromes presenting with lymphatic vessel edema, and a combination thereof.

4.  The use according to claim 1, wherein "promoting the transformation of M1-type macrophages to M2-type macrophages" includes promoting the transformation of M1-type macrophages (promoting inflammatory response) into M2-type macrophages (promoting tissue repair) in myocardial tissue.

5.  Use of TBX1 protein, an encoding sequence thereof, or a detection reagent thereof for preparing a diagnostic reagent or diagnostic kit, wherein the diagnostic reagent or diagnostic kit is used for prognostic assessment of heart diseases associated with cardiac injury or diseases associated with lymphatic abnormalities.

6.  The use according to claim 5, wherein the method for prognostic assessment of heart diseases associated with cardiac injury or diseases associated with lymphatic abnormalities comprises: providing a subject sample, detecting the expression level of TBX1 protein (T1) in the subject sample and comparing it with the average expression level of TBX1 protein (T0) in cardiac lymphatic vessels of myocardial infarction patients;

    if T1 is greater than T0, it indicates a favorable prognosis for the subject;
    if T1 is less than T0, it indicates a poor prognosis for the subject.

7.  The use according to claim 5, wherein the method for prognostic assessment of heart diseases associated with cardiac injury or diseases associated with lymphatic abnormalities comprises: providing a subject sample, detecting whether the subject sample has TBX1 encoding and/or regulatory sequences variants; if the TBX1 variant leads to loss or reduction of TBX1 function, it indicates a poor prognosis for the subject; if the TBX1 variant leads to enhanced TBX1 function, it indicates a favorable prognosis for the subject.

8.  An active ingredient capable of being used for repairing cardiac injuries and treating diseases associated with lymphatic abnormalities, comprising: TBX1 protein, an encoding sequence thereof, or a promoting agent thereof, or a combination thereof.

9.  An expression vector containing an expression cassette for expressing TBX1 protein.

10. The expression vector according to claim 9, wherein the expression cassette has a structure shown in Formula I from the 5' end to the 3' end:

    Z0-Z1-Z2          (I)

    wherein,

each "-" independently represents a chemical bond or nucleotide connecting sequence;
Z0 is absent or a 5'UTR sequence;
Z1 is a nucleotide sequence encoding TBX1 protein; and
Z2 is absent or a 3'UTR sequence.

11. A host cell containing an expression vector according to claim 9 or 10.

12. A pharmaceutical formulation containing (a) the active ingredient according to claim 8, or the expression vector according to claim 9 or 10, or the cell according to claim 11, and (b) a pharmaceutically acceptable carrier, excipient, or diluent.

13. The pharmaceutical formulation according to claim 12, wherein the pharmaceutical formulation further containing: additional active ingredients;
wherein the additional active ingredients comprise: BMP4, CCL28, anti-CD8 antibodies, or a combination thereof.

14. Use of the active ingredient according to claim 8, the expression vector according to claim 9 or 10, the host cell according to claim 11, or the pharmaceutical formulation according to claim 12 or 13, in the preparation of a medication for treating heart diseases associated with cardiac injury and/or diseases associated with lymphatic abnormalities.

15. A method for screening promoting agents of TBX1 protein, which comprises the steps of:
administering a candidate drug to lymphatic endothelial cells cultured *in vitro* and/or experimental animals; if the TBX1 mRNA level or TBX1 protein level in the lymphatic endothelial cells cultured *in vitro* or lymphatic vessels of experimental animals is elevated relative to the control group that was not administered with the candidate drug, or if the function of TBX1 protein is enhanced relative to the control group that was not administered with the candidate drug, then the candidate drug can be used as promoting agent of TBX1 protein.

Figure 1

Figure 2

Figure 3

Figure 4

Figure 5

Figure 6

Figure 7

Figure 8

Figure 9

Figure 10

## INTERNATIONAL SEARCH REPORT

| International application No. |
|---|
| **PCT/CN2021/094932** |

**A.  CLASSIFICATION OF SUBJECT MATTER**

A61K 38/17(2006.01)i;  A61K 48/00(2006.01)i;  A61P 9/00(2006.01)i;  A61P 37/02(2006.01)i

According to International Patent Classification (IPC) or to both national classification and IPC

**B.  FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, VEN, CNTXT, USTXT, WOTXT, EPTXT, CNKI, 万方数据库, WANGFANG, PUBMED, ISI WEB OF KNOWLEDGE: 心肌梗塞, 心肌梗死, 心梗, TBX1, T-box转录因子, 心脏, 心肌, 修复, 先天性心脏病, 先心病, 内皮细胞, 巨噬细胞, 自身免疫, 淋巴管, myocardial infarction, infarct, AMI, cardiac repair, cardiomyocyte, congenital heart disease, macrophage, autoimmunity, lymphatic, lymphangiogenesis, lymphedema, 上海交通大学医学院上海儿童医学中心, 张臻, 张敏, 王文峰, 丁晓宁, 王晔, 杨俊杰, 魏璐

**C.  DOCUMENTS CONSIDERED TO BE RELEVANT**

| Category* | Citation of document, with indication, where appropriate, of the relevant passages | Relevant to claim No. |
|---|---|---|
| X | MARTUCCIELLO, S. et al. "A dual role for Tbx1 in cardiac lymphangiogenesis through genetic interaction with Vegfr3" *The FASEB Journal*, Vol. 34, No. 11, 20 September 2020 (2020-09-20), pages 15062-15079 | 1-15 |
| X | Chen, Li. et al. "Tbx1 Regulates Proliferation and Differentiation of Multipotent Heart Progenitors" *Circ Res.*, 23 October 2009 (2009-10-23), pages 842-851 | 8-12 |
| X | MARTUCCIELLO, S. et al. "Tbx1 interacts genetically with Vegfr3 to regulate cardiac lymphangiogenesis in mice" *BioRxiv*, 18 February 2019 (2019-02-18), pages 1-24 | 1-15 |
| X | Chen, Li. et al. "Tbx 1 regulates Vegfr3 and is required for lymphatic vessel development" *J. Cell. Biol.*, Vol. 189, No. 3, 31 December 2010 (2010-12-31), pages 417-424 | 1, 3, 5-12, 14-15 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **17 November 2021** | **26 November 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2021/094932** |

### C.    DOCUMENTS CONSIDERED TO BE RELEVANT

| Category* | Citation of document, with indication, where appropriate, of the relevant passages | Relevant to claim No. |
| --- | --- | --- |
| Y | Chen, Li. et al. "Tbx 1 regulates Vegfr3 and is required for lymphatic vessel development" *J. Cell. Biol.*, Vol. 189, No. 3, 31 December 2010 (2010-12-31), <br>    pages 417-424 | 1-2, 4-7, 13-14 |
| Y | KLOTZ, L. et al. "Cardiac lymphatics are heterogeneous in origin and respond to injury" *Nature*, Vol. 522, 04 June 2015 (2015-06-04), <br>    pages 62-67 | 1-2, 4-7, 13-14 |
| Y | HENRI, O. et al. "Selective Stimulation of Cardiac Lymphangiogenesis Reduces Myocardial Edema and Fibrosis Leading to Improved Cardiac Function Following Myocardial Infarction" *Circulation*, 12 April 2016 (2016-04-12), <br>    pages 1484-1497 | 1-2, 4-7, 13-14 |
| X | COLE, C.G. et al. "Homo sapiens chromosome 22, GRCh38.p13 Primary Assembly" *Genbank ACCESSION NC_000022 REGION:19756703..19783593*, 01 March 2021 (2021-03-01), <br>    pages 1-12 | 8-12 |
| X | WO 2018098312 A2 (MAYO FOUNDATION FOR MEDICAL EDUCATION AND RESEARCH) 31 May 2018 (2018-05-31) <br>    description, page 1, paragraph 3- paragraph 5, and page 7, paragraphs 3-4 | 1-2, 4-15 |
| X | WO 2020069334 A1 (TERZIC, A. et al.) 02 April 2020 (2020-04-02) <br>    description, page 2, paragraph 1, page 3, paragraph 3, and page 5, last paragraph - page 6, last paragraph | 1-2, 4-15 |
| X | CN 110237250 A (SHANGHAI CHILDREN'S MEDICAL CENTER AFFILIATED TO SHANGHAI JIAOTONG UNIVERSITY SCHOOL OF MEDICINE) 17 September 2019 (2019-09-17) <br>    description, paragraph 19 | 1-2, 4-15 |
| X | CN 110237237 A (SHANGHAI CHILDREN'S MEDICAL CENTER AFFILIATED TO SHANGHAI JIAOTONG UNIVERSITY SCHOOL OF MEDICINE) 17 September 2019 (2019-09-17) <br>    description, paragraph 38 | 1-2, 4-15 |
| A | SANCHEZ-MAS, J. et al. "The TBX1 Transcription Factor in Cardiac Remodeling After Myocardial Infarction" *Rev Esp Cardiol (Engl Ed)*, Vol. Vol. 69, No. No. 11, 30 November 2016 (2016-11-30), <br>    pages 1042-1050 | 1-15 |

**INTERNATIONAL SEARCH REPORT**

| International application No. |
| --- |
| **PCT/CN2021/094932** |

| Box No. I | Nucleotide and/or amino acid sequence(s) (Continuation of item 1.c of the first sheet) |
| --- | --- |

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

### INTERNATIONAL SEARCH REPORT
**Information on patent family members**

International application No.

**PCT/CN2021/094932**

| Patent document cited in search report | | | Publication date (day/month/year) | Patent family member(s) | | | Publication date (day/month/year) |
|---|---|---|---|---|---|---|---|
| WO | 2018098312 | A2 | 31 May 2018 | JP | 2020500857 | A | 16 January 2020 |
| | | | | EP | 3544591 | A4 | 07 October 2020 |
| | | | | US | 2019275071 | A1 | 12 September 2019 |
| | | | | EP | 3544591 | A2 | 02 October 2019 |
| | | | | CA | 3044703 | A1 | 31 May 2018 |
| | | | | WO | 2018098312 | A3 | 05 July 2018 |
| WO | 2020069334 | A1 | 02 April 2020 | None | | | |
| CN | 110237250 | A | 17 September 2019 | None | | | |
| CN | 110237237 | A | 17 September 2019 | None | | | |

Form PCT/ISA/210 (patent family annex) (January 2015)